# EUROPEAN PATENT APPLICATION

(11) **EP 2 975 402 A1**
(43) Date of publication of application: **20.01.2016**
(21) Application number: 14462005.1
(22) Date of filing: 18.07.2014
(51) Int. Cl.: G01N 33/50, G01N 33/68, C12N 5/073

(54) **Viability assessment of in vitro cultured human embryos using protein markers of the culture medium**

(71) Applicant: Pécsi Tudomànyegyetem, 7622 Pécs (HU)
(72) Inventor: Montskó, Gergely, H-7633 Pécs (HU); Kovács, L. Gábor, H-7635 Pécs (HU); Bódis, József, H-7635 Pécs (HU)
(74) Representative: Lengyel, Zsolt

(57) **Abstract**

The non-invasive method of measuring the haptoglobin fragments in human serum albumin supplemented culture medium of in vitro cultured embryos as part of an ART procedure serves as an alternative or supportive method to assess the viability of in vitro cultured embryos without doing any harm to the embryos.

## Description

### Field of the invention

The invention relates to *in vitro* methods for non-invasive embryo viability assessment during assisted reproduction.

### Background art

Infertility affects a relatively high percentage of the mature population leading to an increasing need of the use of assisted reproductive technologies (ART), and in vitro fertilization (IVF). Since the first reported case and introduction of IVF by Steptoe and Edwards (1) the technology became more and more widespread resulting in 125,000 cycles a year in the US (2) in 2007. The total number of ART cycles in Europe was 493,184 in 2007 (3), while in 2008 and 2009 this number reached more than 500,000 cases (4, 5). The development of microsurgical technologies such as the intracytoplasmatic sperm injection (ICSI) means that most couples have the chance of successful fertilization (6). Other improvements like new culture media, advances in cell culturing conditions result in the ability to culture human embryos *in vitro* till even the blastocyst stage is achieved meaning 5-7 days (7, 8). All factors mentioned theoretically suggest a high success rate among IVF experiments in the practice, however, it is far below the expectations. The rate of the successful embryo implantations is surprisingly low (9, 10). In 1995 Dawson et al. (11) reported a success rate of 25%, while in 2004 Scott (12) reported a 27.7% successful delivery rate. In 2009 in Europe this rate went up to 32% depending on the used ART technology (5).

To overcome this limitation of Art, often multiple embryos are transferred in a single cycle (13). Multiple embryo transfer however increases the chance of multiple pregnancies, which has undesired consequences. Multiple gestations can result in the increased risk of preterm delivery accompanied by low birth weight, and the risk of cerebral palsy or lifetime disabilities (12). In 2008 preterm births resulting from multiple pregnancies during IVF caused a 1 billion USD extra cost to the social insurance in the US (13). Due to these considerations the trend is to transfer fewer embryos, though this results in a lower success rate (14), which is the most criticized aspect of IVF (15). According to the present consensus the best option might still be the single embryo transfer if embryo viability could be assessed prior the transfer (16).

The goal therefore is to find reliable morphological and/or biochemical markers to asses the embryo without doing any harm to it and predict the implantation potential. This way we would be able to avoid the health risks of multiple births without sacrificing the chances of a successful pregnancy (17). The easiest and most obvious way to predict embryo viability is by scoring the embryo using morphological markers. These markers include blastomere size, the symmetry and rate of embryo cleavage (18), cumulus-coronal cell morphology (19), cytoplasm granularity and embryo polarity (20). Assessing Morphological markers also include the examination of early pronuclear breakdown (10) and chromosome morphology (21). The main problem with these embryo morphology based techniques is that even embryos described with the best aspect tend to result in unsuccessful pregnancy, or lead to spontaneous abortion in a rate that cannot be explained by maternal reasons alone (22). A possibly better option is the use of molecular markers, such as gene expression profile (6, 23), RNA expression of targeted genes (22, 24) the detection of aneuploidy (25) or the examination of chromosomal complement (21). The other approach is the metabolomic analysis of the embryo using the culturing media, by selecting targeted compounds, or by analyzing the total metabolome (26, 27).

Targeted analysis is conventionally achieved by the measurement of glucose (8) and pyruvate (28, 29) uptake or the measurement of amino acid (30, 31) turnover. Some authors reported that the identification of these parameters resulted in successful prediction of embryo implantation potential, but some studies describe contradictory results. There is a strong consensus that more than one metabolomic parameters should be analysed, and currently the best approach seems to be the measurement of the total metabolome (32). Analytical tools cover near infra-red spectroscopy (33), Raman spectroscopy (2), and more recently mass spectrometry (22, 34, 35). Mass (MS) spectrometry has the potential of specific and sensitive quantification in a wide spectrum of molecular mass ranges and therefore suites well the needs of metabolomic or proteomic fingerprinting and quantification.

According to the current protocols, the viability of the embryo during the IVF process is assessed by microscopic inspection, resulting in a pregnancy rate of 25-30 %.

Haptoglobin (Hp) is a tetrameric protein secreted by the liver and involved in binding free haemoglobin. The mature protein molecule consists of two disulfide linked α and β chains. The chains originate from a common precursor protein, which is proteolytically cleaved during protein synthesis.

Haptoglobin has been found in all mammals studied so far and in its simpler form, in bony fishes. It appears to be present in some birds, but being absent from neognathous birds and from at least some amphibians (Xenopus).

The quaternary structure of haptoglobin in organisms other than humans typically consists of a dimer of alpha-beta-chains covalently linked by a disulphide bridge between the Cys15 residue of each alpha-chain. However, in humans the βchains are identical in all haptoglobin types (glycosylated 40 kDa, 245 amino acids; unglycosylated 35 kDa) while the α chains have two allelic forms in humans, alpha-1 and alpha-2, determining three possible genotypes. When haptoglobin is synthesized as a pre-protein it is cleaved by the endoplasmatic reticulum to the mature peptide containing two alpha and two beta chains connected by a disulphide bond (40). Haptoglobin can be fragmented into the alpha and beta chains by the cleavage of the disulphide bond via cleaving enzymes or chemical reduction.

Human haptoglobin is known to accumulate in HSA (human serum albumin) standards used in cell culture media during HSA purification (38). The human haptoglobin alpha-1 chain is a 9186.5Da protein present in HSA supplemented cell culture medium used for culturing human embryos.. The source of the haptoglobin alpha-1 fragment in the HSA standard and finally in the culture medium can be the cleavage of the disulphide bond connecting the alpha and beta chains of the mature haptoglobin-1 molecule (39).

### Summary of the invention

The present invention is aimed at providing a more accurate non-invasive embryo viability assessment by quantitation of the alpha-1 fragment of human haptoglobin, which was found to be present in a significantly greater amount in the culture medium of non-viable than of viable embryos cultured *in vitro* as a part of the ART process. According to our measurements in the group of culture media of embryos, which were assigned in the biochemical assay as non-viable (based on the amount of the haptoglobin fragment) there were no pregnancies detected, thus this assay revealed a 100% successful selection of the non-viable embryos. In the group assigned as viable, the rate of birth was 54%.

In an aspect the invention relates to an *in vitro* method for non-invasive embryo viability assessment comprising
a) providing a test sample of an embryo culture medium to obtain a test sample;
b) providing a sample of a control culture medium without the embryo to obtain a control sample;
   both the embryo culture medium and the control culture medium comprising haptoglobin,
c) detecting the level of a fragment of haptoglobin in the test sample and in the control sample;
wherein a higher level of said haptoglobin fragment in the test sample as compared to the control sample is indicative of an embryo in the embryo culture medium, said embryo having a limited chance for or being inappropriate for resulting in successful pregnancy.

Thereby those embryos having a limited chance for or being inappropriate for resulting in successful pregnancy, may be considered as non-viable and can be selected out from a set of embryos and the rest of them may be considered as viable for the object of the present invention (unless considered as non-viable by any other method). Thereafter preferably only an embryo considered as viable may be used for implantation. Thereby the rate of successful pregnancy is significantly higher than without pre-selection.

In a preferred embodiment the fragment of the haptoglobin molecule is a reduced haptoglobin fragment obtained by reduction of a disulphide bond between alpha and beta chains of haptoglobin, said fragment having a sequence specific for said haptoglobin chain.

Preferably, the fragment of the human haptoglobin is selected from a haptoglobin alpha fragment and a haptoglobin beta fragment. Preferably the haptoglobin alpha fragment is a haptoglobin alpha-1 fragment.

In an embodiment, in the method according to the invention detection of said fragment of haptoglobin is carried out by an assay method selected from the group consisting of
- mass spectrometry,
- immunoassay,
- a spectroscopic method
- a functional test,
- a sequencing method or a combination thereof.

Any method used for detection should preferably be specific for the fragment of haptoglobin the level of which is to be detected.

Preferred methods are mass spectrometry and immunoassays.

Immunoassays include any method wherein an epitope-specific binding molecule is applied to detect the level of the fragment of haptoglobin according to the invention. Such epitope-specific binding molecules include antibodies e.g. monoclonal antibodies and antibody fragments and molecules having a binding region appropriate for binding to an epitope, like minibodies and antibody mimetics, like affibodies, anticalins, monobodies etc.

Preferred assays are ELISA, Western-blot etc.

In a preferred embodiment a separation step of separating said fragment from the test sample and from the control sample is carried out before the detection of the fragment. This separation step also can be utilized to make the method fragment-specific, if resolution of the separation is sufficiently high to separate the haptoglobin fragment to be detected from other haptoglobin fragments or proteins interfering with the measurement.

Separation can be performed based on any molecular property in which the fragments differ and which is suitable to achieve a sufficient separation, like size, charge, hydrophobicity, isoelectric point, affinity, etc.

For example in a Western-blot experiment an electrophoretic step may precede the blotting step resulting in a separation of the fragments.

In a preferred embodiment of the method if the level of the haptoglobin fragment in the test sample is higher than that of the control sample and is equal at least with a threshold value, preferably a pre-determined threshold value, this fact is indicative of a limited chance of the embryo for or being inappropriate for resulting in successful pregnancy.

In a preferred embodiment of the method if the level of the haptoglobin fragment in the test sample is at least 110%, 115%, 120%, 125%, 130%, 135% or 140% of that of the control sample, the embryo is classified non-viable. Optionally, if the level of the haptoglobin fragment in the test sample is under 110%, 115%, 120%, 125%, 130%, 135% or 140% of that of the control sample the embryo is classified viable.

In a preferred embodiment the threshold value is pre-determined experimentally.

In an embodiment levels in case of embryos resulting in birth is compared with levels in case of embryos which do not result in birth.

In an embodiment, the threshold is set to a value wherein embryos for which the level is lower than the threshold do not result in birth in 100% of the implantations (cycles).

In an other embodiment, the threshold is set to a value wherein embryos for which the level is at least equal to the threshold result in birth in at least 40%, 50%, 60%, 70%, 80% or 90% of the implantations (cycles).

In a preferred embodiment the embryo is a mammalian, preferably a human embryo. Highly preferably the embryo is a human embryo.

In a preferred embodiment the haptoglobin is a mammalian, preferably a human haptoglobin. Highly preferably the haptoglobin is a human haptoglobin.

In a preferred embodiment the embryo is a human embryo, the haptoglobin is a human haptoglobin, and the haptoglobin fragment is selected from a group consisting of a haptoglobin beta fragment, haptoglobin alpha fragment, haptoglobin alpha-1 fragment, haptoglobin alpha-2 fragment, a haptoglobin alpha-beta fragment, haptoglobin alpha-1-beta fragment, haptoglobin alpha-2-beta fragment; preferably from a group consisting of a haptoglobin beta fragment, haptoglobin alpha-1 fragment, haptoglobin alpha-1-beta fragment, preferably from a haptoglobin alpha-1 fragment or a fragment comprising multiple fragments, however, less fragments than the whole haptoglobin..

In a preferred embodiment both the sample and the control culture media comprise serum albumin.

In a preferred embodiment the non-invasive embryo viability assessment method of the invention is combined with an other non-invasive embryo viability assessment method. Preferably, if the embryo is assessed to be non-viable based on any of the methods it is not selected for implantation. Preferably, if the embryo is assessed to be viable based on each of the viability assessment methods, it is selected for implantation. Preferably, the method for non-invasive embryo viability assessment is combined with the microscopic evaluation of embryo viability based on embryo morphology.

In a further aspect the invention also relates to a method for in vitro fertilization of a female patient, wherein the method comprises the embryo assessment method of the invention, and the additional step of implanting at least one embryo into the uterus of a female patient. Preferably, the female patient is a fish, bird or a mammal having haptoglobin. More preferably, the female patient is a mammal. Even more preferably the female patient is a human.

In a further aspect the invention also relates to a use of an embryo culture medium for non-invasive embryo viability assessment by measuring the level of a haptoglobin fragment as defined herein, said medium comprising haptoglobin.

In a further aspect the invention also relates to the use of a haptoglobin fragment as defined herein for non-invasive embryo viability assessment.

In a further aspect the invention also relates to the use of a detectable binding molecule capable of specifically binding to a fragment of a haptoglobin as defined herein for non-invasive embryo viability assessment.

Preferably non-invasive embryo viability assessment is carried out by a method for non-invasive embryo viability assessment as defined herein.

In a further aspect the invention relates to kits for determining the level of a haptoglobin fragment containing ready-to-use immunoassay(s) containing an epitope-specific binding molecule for detecting the level of a fragment of haptoglobin according to the invention.

In an embodiment a kit contains culture medium suitable for embryo culturing with a predetermined haptoglobin content.

In a preferred embodiment the kit contains culture medium suitable for embryo culturing with a predetermined haptoglobin content and a ready-to-use immunoassay as defined above.

In a highly preferred embodiment the kit is in the form of a chip suitable to carry out various biochemical measurements simultaneously.

### Definitions

The term "embryo" as used herein refers to an embryo, preferably a human embryo in the first stage of development, between the fusion of gametes and the blastocyst stage. The term "embryo" as used herein refers to the morula stage of the development of an embryo. Also, the term "embryo" as used herein refers to an in vitro fertilized and cultured embryo prior to implantation.

The term "viable" when used herein referring to an embryo refers to an embryo of which the in vitro culture medium contains a level of haptoglobin below a pre-determined threshold indicating that the embryo is not impaired and may be appropriate for implantation. Preferably, the in vitro culture medium of the embryo contains less than 110%, 120%, 130%, 140% or 150% of the amount of a haptoglobin fragment of the control culture medium not comprising the embryo. Nevertheless, Tthe implantation of a viable embryo into the uterus of a female patient may or may not result in pregnancy and/or may or may not result in successful delivery, as these results depend on many other factors.

"Haptoglobin" is a multimeric protein having subunits linked by disulphide linkages, and being expressed as a precursor polypeptide chain (zonulin) and processed into alpha and beta chains. Haptoglobin belongs to the peptidase S1 family and contains a peptidase S1 domain and sushi (CCP/CCP) domains. Preferably haptoglobin according to the invention shows at least 30%, 40%, 50%, 60%, 70%, 80% or 90% sequence identity with a fish, bird or mammalian, preferably mammalian haptoglobin sequence e.g. a human haptoglobin sequence. Mammalian sequences are given e.g. in the UniProtKB/Swiss-Prot database, e.g. on the following reference numbers: P00738; G3RSR8; J3QR68; H2RAT6; G3RB80; P00738-2; Q28800; Q28803; G3S6B1. Haptoglobin is encoded by the HP gene (in humans) or a gene corresponding thereto. Preferably native haptoglobin functions to bind free plasma haemoglobin, what allows degradative enzymes to gain access to the haemoglobin while at the same time preventing loss of iron through the kidneys and protecting the kidneys from damage by haemoglobin.

A "haptoglobin fragment" is a part of the haptoglobin molecule consisting of a shorter chain and/or a subunit thereof or both.

A "reduced fragment" of a multi chain protein is obtained by reduction of a disulphide bond between chains of said protein, said fragment having a sequence specific for said chain. Thus, a "reduced haptoglobin fragment" is a fragment obtained by reduction of a disulphide bond between subunits of the haptoglobin, said fragment having a sequence specific for said haptoglobin chain.

In the present invention mutated haptoglobin subunit, fragment or chain sequences are contemplated provided that their sequence is at least 30%, 40%, 50%, 60%, 70%, 80% or 90% identical with native fish, bird or mammalian sequences calculated with reference to the full length of the native sequence of the subunit, fragment or chain, and wherein with a detection method such mutated sequence is detected together with the native subunit, fragment or chain. Thus, a mutated sequence is to be understood as including any difference including substitutions, deletions and optionally additions. Thus, fragments having a shorter chain length are contemplated so far they can be detected together with and/or specifically to the native subunit, fragment or chain.

The "haptoglobin alpha-1 chain" refers to the full length chain of a haptoglobin alpha-1 subunit. The terms "haptoglobin alpha-1 fragment" and "haptoglobin alpha-1 chain" are used interchangeably. The term "haptoglobin alpha-1 fragment" may be understood as a "haptoglobin alpha-1 chain" and, as used herein, also may refers to any fragment of the haptoglobin alpha-1 chain, that has an amino-acid sequence specific to said chain. This refers to mutates mutandis to other subunits.

A "sample" of a substance, e.g. culture medium means a part or a whole (e.g. whole part) of said substance which is appropriate for handling in an experiment or assay.

As used herein, in a broader sense, a "part" of an entity is understood as also including the whole entity unless otherwise specified, whereas said entity is composed of one or more parts. A fragment is a part which does not involves the whole. In a narrower sense a part is a fragment of the whole entity. Preferably, a fragment is a part which is specific to the whole entity. In an aspect one or more parts make up the whole entity.

A "culture medium" as used herein is a substance useful and appropriate for culturing cells, either separate cells or cells attached to each other or a tissue. In a preferred embodiment the culture medium is an embryo culture (or culturing) medium appropriate for culturing an embryo, preferably from the one cell stage to the blastocyst stage. Preferably the embryo is a fish, bird or mammalian embryo, preferably a mammalian embryo, highly preferably a human embryo.

A "higher level" of a substance in a first sample than in a second sample indicates that the level, depending on the method for measuring and, if appropriate, calculating said level, including optionally the statistical method involved, is noticeably, detectably or significantly higher in the first sample than in the second sample. Preferably "higher level" means that the level is above or is at least equal to a threshold value, e.g. as defined herein.

"Detection" involves a technical method wherein a substance is detected in a sample and includes or comprises quantitative or semi-quantitative detection; thus, the term can be understood as including measuring, assessment and/or calculation steps or a combination thereof as required to obtain the desired information. Thus, in a preferred version, detecting means measuring and/or assessing.

As used herein the singular forms "a", "an" and if context allows "the" include plural forms as well unless the context dictates otherwise.

The term "comprises" or "comprising" or "including" are to be construed here as having a non-exhaustive meaning and allow the addition or involvement of further features or method steps or components to anything which comprises the listed features or method steps or components.

The expression "consisting essentially of" or "comprising substantially" is to be understood as consisting of mandatory features or method steps or components listed in a list e.g. in a claim whereas allowing to contain additionally other features or method steps or components which do not materially affect the essential characteristics of the use, method, composition or other subject matter. It is to be understood that "comprises" or "comprising" or "including" can be replaced herein by "consisting essentially of" or "comprising substantially" if so required without addition of new matter.

### Brief description of the figures

**Figure 1** Mass spectrum of the identified haptoglobin fragment. The most intensive ion at m/z 1149.7 m/z corresponds to the [M+8H]⁸⁺ ion of the molecule. The ones at m/z 1022.1 and m/z 1313.7 are the other two multiply charged ions [M+7H]⁷⁺ and [M+9H]⁹⁺ detected with acceptable signal to noise ratio.
**Figure 2** Results of the blinded analysis of embryo culture medium after three days of incubation (n=80).
**Figure 3** Bar chart of the mean amount of the haptoglobin alpha-1 fragment amount in the control (n=12), viable embryo and non-viable embryo samples (total n=90). A significant difference (p<0.001) was found between the non-viable embryo and the other two groups.

### Detailed description of the invention

Viability assessment prior embryo transfer is a crucial question since the success rate of IVF interventions is surprisingly low worldwide. The practice of multiple embryo transfer to overcome this limitation is accompanied by several risk factors leading to a consensus that the best option is the single embryo transfer (32) where the implantation potential of the embryo could be assessed *in vitro* during the first couple of days of development. Rather than the routinely used embryo morphology based approaches like cleavage rate, or blastocyst symmetry, measurement of the metabolomic processes of the developing embryo, or the search for biomarkers (37) in the cell culture medium seems to be a better option. The aim of our work was to find any biomarker present in the embryo culture medium using mass spectrometry, which would qualitatively or quantitatively differ in the samples of viable and non-viable embryos.

We have unexpectedly found that the amount of a fragment of a polypeptide present in the culture medium conventionally used for culturing embryos as part of the IVF process has increased in the culture media of embryos that later proved to be non-viable, i.e. the implantation of which did not result in successful delivery, compared to the amount of the same fragment measured in the culture media of embryos proved to be viable and in control culture media without an embryo. We identified the said fragment as the human haptoglobin alpha-1 fragment, derived from the protein haptoglobin by way of the cleavage of the disulphide bond connecting the alpha and the beta chain of the haptoglobin molecule.

During pre-implantation the number of embryonic cells increases; however this increase is the result of cell proliferation and cell death. In contrast to normally developing embryos the ones with lower viability show a higher rate of apoptosis similar to the arrested embryos, finally resulting in secondary necrosis and an increase in membrane permeability (35, 36). Apoptosis involve the activation of proteases and other enzymes, which due to membrane disintegration might be released into the culturing medium. When cell membrane disintegration accompanying cell death happens, these activated proteolytic enzymes leave the cell and start cleaving proteins found in the culture media.

When haptoglobin is synthesized as a pre-protein it is cleaved by the endoplasmatic reticulum to the mature peptide containing one alpha and one beta chain connected by a disulphide bond (40). In a reducing chemical environment this bond can break, forming two sulphydryl groups. The explanation for the increased amount of this alpha-1 fragment in the samples of unsuccessful embryos might be the fact that abnormally developing embryos often show the characteristics of apoptosis in a larger extent than normal embryos, later followed by secondary necrosis accompanied by increased membrane permeability (36).

We suggest that during its development the non-viable embryo changes the chemical composition of the culture medium to a more reducing environment, thus causing the increased cleavage or reduction of the disulphide bond connecting the alpha and beta chain of haptoglobin. The processes of apoptosis and finally necrosis accompanied by an increase in membrane permeability and the changes in the chemical composition of the culture medium may result in the increase of the amount of several polypeptide fragments cleaved by the activated proteolytic enzymes from proteins of the culture medium. This may happen only in the culture media of abnormally developing embryos as the level of apoptosis is lower in normally developing embryos. Along with the amount of the alpha-1 fragment of the human haptoglobin protein, the amount of the β chain or the amounts of shorter fragments specific to this protein may increase. The fact that disulphide bond cleavage is the source of the haptoglobin alpha 1 fragment was confirmed in our experiment where the disulphide bond reducing agent dithiothreitol was added to the control (no embryo containing) G1+HSA medium and an increase was observed in the amount of the alpha-1 fragment. The presence of free thiol groups was confirmed by the addition of 2-iodoacetamide, an alkylating agent of thiol groups.

in the light of the above described process of apoptosis observed when an embryo develops abnormally, we suggest that proteins of the culture media of a embryo cultured in vitro as part of an IVF process, and in particular haptoglobin are fragmented by cleaving enzymes or chemical reduction and these fragments can be detected, quantified and thus used as indicators of abnormal development. Such fragments can be identified prior to the implantation of the embryo from a sample of the culture medium, without doing any harm to the embryo, by methods well known in the art, e.g. by HPLC. The amount of such fragments can be quantified and compared with the amount of fragments in a control sample likewise by methods well known in the art, such as mass spectrometry or various immunoassays.

The vast majority of currently used in vitro embryo culturing media contains serum proteins as these molecules are a crucial, seemingly irreplaceable component. In case of human embryos, the serum proteins used are conventionally proteins derived from human serum. Macromolecular supplementation of culture media, in the form of whole serum, isolated serum proteins, recombinant serum proteins, or alternative polymers provides both chemical and physical interface of embryos with their microenvironment and are crucial for maximizing embryonic viability (41). The role of macromolecular supplementation of the human embryo culturing media is described in detail by e.g. Patrick Quinn: Culture Media, Solutions, and Systems in Human ART, Cambridge University Press, 2014.(41) and in Manual of Assisted Reproductive Technologies and Clinical Embryology, JP Medical Ltd, 2012 (42).

The concern regarding diseases transmissable by the use of protein obtained from donors (e.g. prion-associated diseases) in the culture media of human embryos has lead to several attempts to substitute donor derived serum and serum proteins and also to the development of protein free media (PFM). The use of recombinant proteins (e. g. albumin) has been suggested (43). EP 2 235 160 describes a substantially protein free culture medium to be used in in vitro fertilization and other assisted reproduction technologies in humans. However, the use of these alternative media has remained marginal to the date of this application.

Conventionally used embryo culturing media contain human serum albumin (HSA) as a growth promoting agent. HSA is most often purified from donor serum and it is a described fact that during purification several other polypeptides such as haptoglobin can accumulate in commercially available standards (38). We have investigated how different batches of the HSA standard affect the initial haptoglobin alpha-1 fragment content of the medium. The variation found was much lower than the difference expressed as a percentage between the viable and non-viable embryo groups.

During the discovery phase of our study (n=10) a novel polypeptide marker was found which showed a significant differences (p<0.001) in quantity between the successful and unsuccessful embryo groups. This molecule was identified with tandem mass spectrometry as the alpha-1 fragment of human haptoglobin. Detection and quantitation of the alpha-1 haptoglobin fragment of the culture medium proved to be a good additional method to the conventionally used microscopic inspection. The method of the invention identifies non-viable embryos which - in spite of their promising microscopic morphology - should not be implanted. Using the method of the invention, We were able to select with 100% accuracy the embryos with zero potential to result in pregnancy. In this group the observed increased amount of the 9186.5 Da haptoglobin fragment proved to be a quantitative indicator of negative prediction prior embryo transfer. The combination of microscopic evaluation with the measurement of the haptoglobin fragment increases the success rate to 50 %.

According to our results, the determination of the amount of the human haptoglobin alpha-1 fragment in a sample of the culture media of embryos prior transfer and comparing the amount of said fragment to blank control samples incubated together with the embryos under the same circumstances may provide a more accurate non-invasive embryo viability assessment to increase the success rate of IVF processes.

Detection of the amount of fragments of the haptoglobin molecule in samples of the culture media may be achieved by various protein/peptide detecting methods known in the art. Such methods include e. g. spectrometric and spectroscopic procedures, such as mass spectrometry.

For example, MALDI-TOF mass-spectrometry has become a popular tool. High throughput and relative simplicity of this technology have made it attractive for biomarker discovery and validation. Furthermore, technical approaches have been developed for protein-chip based SELDI-TOF mass-spectrometry (46), see US 2003/0017515A1. Such methods can be accompanied by purification techniques to enrich the sample in the haptoglobin fragment. Such techniques involve purification with magnetic beads, chromatographic methods, such as liquid chromatography, etc. In mass spectrometry peak area may correlate with concentration.

A further preferred option involves different immunoassays, such as Western blot, radio-immune assay, ELISA, sandwich enzyme-linked immunosorbent assay, etc.

Immunoassay typically use antibodies. In the present invention, analogously, other binding molecules like antibody mimetics can be used. A requirement for antibodies and binding molecules like antibody mimetics to be used in the present invention is that it should be capable of recognizing the haptoglobin fragment to be detected and differentiate it from the whole haptoglobin or a fragment thereof which is not to be detected in the assay setup. For example binding molecules like antibodies specific against the alpha or beta chains can be used (47).

Ready-made antibodies against both the alpha and the beta chains are commercially available for the above mentioned detection methods. Antibodies against the intact haptoglobin molecule are also available commercially (Abcam, Randox LifeSciences, Santa Cruz Biotechnology, Acris Antibodies GmbH etc.). The combined use of an antibody against either the alpha or the beta chain and against the intact haptoglobin may enhance the accuracy of the quantification measurements. Manifacturing of tailor made antibodies is also extensively described in the literature and is well within the knowledge of the skilled artisan.

A specific detection of Haptoglobin-alpha 1 Subunit is described e.g. in US 2003/0017515 A1 and in (49)

A detailed description of the techniques which may be used when carrying out the method of the invention is found e.g. in The Immunoassay Handbook, Fourth Edition: Wild D: Theory and applications of ligand binding, ELISA and related techniques Elsevier Science; 4. edition, 2013 and in Gosling, JP Immunoassays: A Practical Approach Oxford University Press; 2000 (44 and 48).

These methods may be combined with fast sequencing method allowing a specific detection of antibody chains.

Thus, such antibodies or binding molecules can be used in kits for assessment of viability of embryos for the purpose of in vitro fertilization methods. Such a kit may also comprise a culture media or components thereof for culturing embryos as well as control media, haptoglobin standard or compositions comprising such haptoglobin standard. Haptoglobin standard preferably comprises a fragment thereof to be detected and/or other fragments or haptoglobin itself as controls which are not to be detected. Means for separation of haptoglobin fragments may be included as well.

It is possible to use the method of the invention even in ART processes where the culture medium is supplemented with non-serum derived albumin or the culture medium is a protein free medium. Haptoglobin may be added to such media after fertilization as a biochemical marker without the risk of interfering with the development of the embryo.

### Examples

### MATERIALS AND METHODS

### Patients

Our study was performed between March 24, 2013 and May 9, 2013 in the Assisted Reproduction Unit, Department of Obstetrics and Gynecology, University of Pécs, Hungary. In this period we started 90 unselected IVF cycles and we made transvaginal ultrasound guided aspiration of follicular fluid. The patients participated in our IVF program were aged 23-40 years (mean: 32.3±5.1 years) and had BMI of 21.3-29.7 (mean: 23.80±1.9). They presented with the following main infertility diagnosis: male factors (14, 33.3%), damaged or blocked Fallopian tubes (10, 23.8%), severe endometriosis (7, 16.7%) and unexplained infertility (11, 26.2%). These latter patients experienced six unsuccessful intrauterine inseminations previously. The patients were collected into this study according to the date of the procedure, so it was an unselected population. Among the patients there were no individuals with known diabetes mellitus (type I and II), or reduced glucose tolerance.

### Study Protocol

GnRh agonist triptorelin (Gonapeptyl; Ferring^{®}, Germany) was used in a long or short protocol, and the stimulation was performed with individual dosages of rFSH (Gonal-F; Serono^{®} Aubonne, Switzerland), varying from 100 to 225 IU per day depending on the follicular maturation. The starting dose was adapted according to the BMI and the age. For patients with a previously known low response it was increased to a maximum dose of 300-350 IU daily. The follicular maturation was determined by ultrasound examination from the 6th day of the cycle, every other day. We changed the amount of the administered gonadotropins individually according to the size of the follicles. Ovulation was induced by injection of 250 ug of hCG (Ovitrelle; Serono^{®}Aubonne, Switzerland) when at least two follicles exceeded 17 mm in diameter, and aspiration of follicular fluid was performed 36 hours later by ultrasonography-guided transvaginal puncture under routine intravenous sedation. The oocyte collection was performed using a Sonoace 6000C two dimensional real time ultrasound scanner equipped with 4-8 MHz endovaginal transducer. The oocyte collection was performed in G-MOPS^{™} medium (Vitrolife, Göteborg, Sweden).

### Fertilization methods

We performed the fertilization with Intracytoplasmatic Sperm Injection (ICSI) depending on the andrological status (sperm count less than 15 M/ml), the maternal age (> 35) and the number of the previous IVF cycles the patient had before (>2). The oocytes selected for ICSI were denuded with hyaluronidase and were assessed for maturity. Only metaphase II oocytes, identified by the presence of the first polar body, were chosen for fertilization. ICSI was performed 3-6 h after oocyte recovery in the medium G-MOPS^{™}. The remained oocytes were fertilized with the conventional IVF method in a bicarbonate buffered medium (G-IVF^{™}, Vitrolife^{®}, Göteborg, Sweden). Fertilization was assessed 24 hours later in the medium G-1^{™}v5 (Vitrolife^{®}, Göteborg, Sweden).

Embryo transfers were done 3 or 5 days after the oocyte retrieval. From day 3 to blastocyst stage we use the medium G-2^{™}5 (Vitrolife^{®}, Göteborg, Sweden) supplemented with human serum albumin (HSA, Vitrolife^{®}, Göteborg, Sweden) in a 5 mg/ml concentration. According to the patient request we transfer one, two or three embryos. Cryopreservation of the remaining embryos was performed at this stage according to the Hungarian law. Progestogen supplementation was provided using 300 mg of progesterone 3 times a day (Utrogestan; Lab.Besins International S.A.^{®}, Paris, France). To evaluate the success of the treatment transvaginal ultrasound examination was performed 21 days after the embryo transfer to detect gestational sac. After the transfer culture medium samples were kept at -24C in 40µl aliquots until the measurement.

### Solvents and internal standard preparation

All solvents used for internal standard solution and HPLC solvent preparation were of LC-MS grade, purchased from Molar Chemicals (Molar Chemicals, Hungary). Water, acetonitrile, and formic acid were used in the HPLC solvents.

Cortisol (Sigma-Aldrich, Budapest, Hungary) was used as internal standard; a 3.86 µm/L solution was prepared in 20% methanol. After thawing the culturing media solutions, to every 25 µl of media 5 µl of IS solution was added and was vortex mixed for five seconds. The injection volume was 20 µl.

### LC-MS conditions

A Dionex Ultimate 3000 (Dionex Corp., USA) analytical HPLC equipped with an autosampler and a column thermostat set at 30ºC was used. Separation was carried out on a Kinetex C18 2.6 µm, 2.1 x 100 mm analytical column (Phenomenex, USA) with a multi-step gradient elution at a flow rate of 200 µL/min. HPLC solvents contained 0.1% formic acid in 5% (A) and 95% (B) acetonitrile in water. The gradient profile is described in Table 1; the total runtime is 27 minutes.

**TABLE 1 Description of the HPLC gradient used in the LC-MS run.**

| Time (minute) | A% | B% |
|---|---|---|
| 0.0 | 100 | 0 |
| 16.0 | 60 | 40 |
| 20.0 | 0 | 100 |
| 21^{a} | 100 | 0 |

| | | |
|---|---|---|
| ^{a} At the end of every chromatographic run a six minute re-equilibration phase was inserted to start the new run at 100% "A" solvent concentration. | | |

The mass spectrometer coupled to the HPLC was a Bruker micrOTOF accurate mass instrument equipped with an electrospray ionisation source (ESI) operated in the positive mode. Main source parameters were: capillary voltage: 4500V, nebuliser pressure: 2.4 bar, drying gas (N₂): 8 L/min and drying temperature: 210ºC. Mass spectra were collected between 500 m/z and 1500 m/z. Internal mass calibration was performed at the beginning of every run using the peaks of Na⁺ formate clusters.

### Statistics

All statistical analysis was made using the IBM SPSS Statistics Version 20 (IBM Magyarország Kft. Budapest, Hungary) software. Normality of the dataset was investigated using the Kolmogorov-Smirnov test, while the differences in the amount of the peptide fragment between the experimental groups (incubated blank medium, successful and unsuccessful embryos) by analysis of variance (ANOVA) and Student's t-test.

### Example 1. Identification of proteins in the culture medium

Unused culture medium was chromatographed on Aeris Peptide HPLC column (2.1 x 150 mm) using gradient elution and fractions were collected. Fractions containing proteins of interest were constructed of 10 consecutive runs and were lyophylized. Protein(s) in this pooled sample was identified by proteomics method. Disulfide bridges were reduced (DTT) and alkylated (iodoacetamide) and polypeptides were digested with trypsin [1]. Peptides in the reaction mixture were separated by nanoHPLC on an EASY-column (75 µm x 100 mm, C18-3 µm, Thermo Scientific) and analyzed by an online connected a MAXIS 4G QTOF mass spectrometer equipped with captive spray ESI ionsource (Bruker, Bremen). The mass spectrometer was operated in data dependent analysis (DDA) cycling mode to automatically collect MS/MS fragmentation spectra of the 3 most abundant ions in every MS scan. Raw data files were processed and MS/MS peaklists were produced in Bruker DataAnalysis 4.0. Peaklists were submitted for protein identification to Mascot 2.4 (Matrixscience,) using Bruker Proteinscape 2.1. Swissprot database (version 2013.10, containing 541,561 sequences) was used for identification. Additional searches were performed on an in house created database, which contained all variants of HPT_HUMAN and HPTR_HUMAN proteins. Search parameters used in all cases for identification were: 120 ppm and 0.25 Da precision for parent mass and fragment spectra, respectively. Trypsin as digestion enzyme (assuming max. 2 missed cleavage sites), carbamidomethylation of cysteins as fixed, and oxidation of methionines as variable modifications were used for data analysis. Identified MS/MS spectra were manually validated using Bruker Biotools 3.2 software.

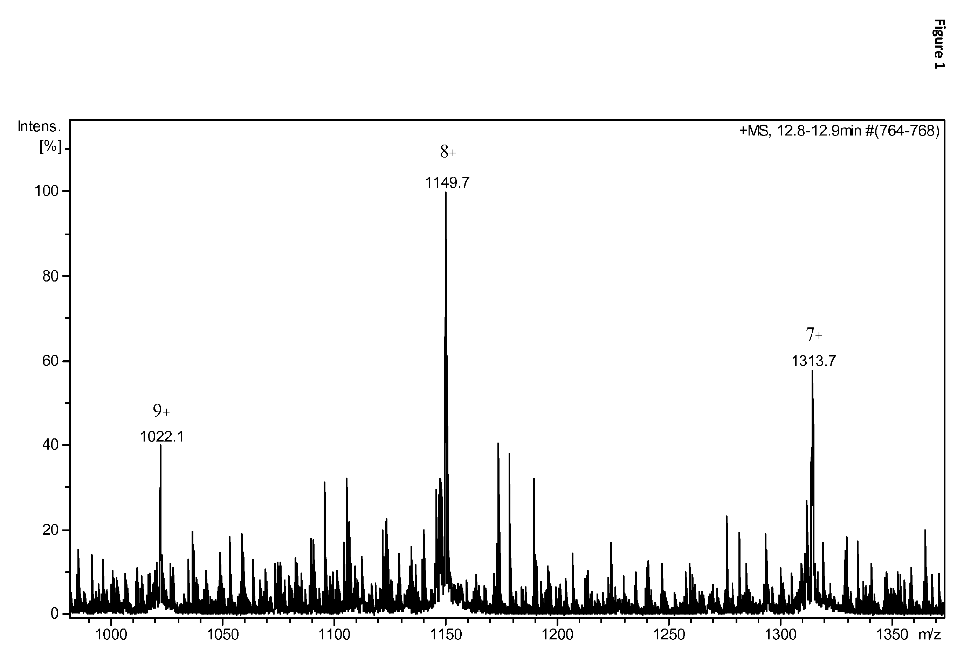

Chromatographic conditions of the applied gradient are summarized in Table 1. Our primary aim was to find any qualitative or quantitative difference between incubation media of successful and unsuccessful embryos. This was done by measuring a smaller number of culture media samples of embryos transferred on day 3 (n=10) and embryos transferred on day 5 (n=10), all with known clinical outcome. Half of the samples were from successful and half from unsuccessful embryos. As control, empty incubated G-1^{™}v5 (3 day transfer) or G-2^{™}v5 (5 day transfer) containing 5 mg/ml HSA was used (n=4). The incubation of the control samples were done parallel with the embryos under the same circumstances and time interval. Qualitative differences were not found, however four different polypeptides were detected which all notable differed in quantity between the two embryo groups. These molecules were also present in the blank control samples however absent from the G-1^{™}v5 and G-2^{™}v5 containing no HSA. The compounds were detected at m/z 798.9 [M+6H]⁶⁺, m/z 745.1 [M+6H]⁶⁺, m/z 771.9 [M+6H]⁶⁺ and m/z 1149.3 [M+8H]⁸⁺. Deconvolution of the obtained mass spectra revealed that the monoisotopic masses of the four molecules are 4787.4 Da, 4464.6 Da, 4622.4 Da and 9186.5 Da respectively. Quantification was done using the peak areas (without unit of measurement) of the corresponding ion chromatographic peaks. Peak areas of the detected compounds were standardized using the summarized peak areas of the H⁺ and Na⁺ adduct ions of the IS cortisol. We found that the compounds were present in the samples of unsuccessful embryos in a 30-60% larger extent than in the samples of the successful embryos, or the control samples. No notable difference was observed however between the control samples and the samples of the successful embryos. Similar observation was made in the case of 3 day and 5 day embryos, though in the latter case the difference was only 10-30% depending on the selected compound. To find out whether the observed difference was significant, Student's t-test was performed which revealed that only the 9186.5 Da molecule differs significantly in its amount between the two embryo groups (p=0.005). For this reason we further concentrated only on the 9186.5 Da molecule (Fig. 1).

The next step was the MS/MS identification of the polypeptide according to the protocol described in the materials and methods section. Database search using MS/MS data identified two peptide fragments assigned to human haptoglobin. According to manual investigation of sequence annotations of database entries and literature data the protein of interest identified by the two MS/MS fragments proved to be the haptoglobin alpha-1 chain. This form of haptoglobin alpha-1 subunit has a monoisotopic mass of 9186.4; all identified sequences correspond to this region of the haptoglobin precursor protein. The overall sequence coverage of the identification was 62%. The complete sequence of the haptoglobin alpha-1 fragment with the two identified MS/MS fragments with significant hits is summarized in Table 2.

**TABLE 2 Identification of peptides assigned to haptoglobin alpha-1. Underlined sequences correspond to the two significantly identified MS/MS fragments of the polypeptide. Identification was carried out on an in-house built database of human haptoglobin variants.**

| P00738 (HPT_HUMAN) Haptoglobin VAR_017112 [19-102] (Haptoglobin alpha-1) | | | | | | | | |
|---|---|---|---|---|---|---|---|---|
| Sequence | | | Mass | | | Mascot Score | Expect Value | Peptide Sequence |
| Start | - | End | Observed | Mr(expt) | Mr(calc) | | | |
| **VDSGNDVTDIADDGCPKPPEIAHGYVEHSVRYQCKNYYKLRTEGDGVYTLNDEKQWINKAVGDKLP ECEAVCGKPKNPANPVQ** | | | | | | | | |
| 42 | - | 54 | 720,9006 | 1439,787 | 1439,642 | 88 | 1,50E-09 | R.TEGDGVYTLNDEK.Q |
| 60 | - | 76 | 620,043 | 1857,107 | 1856,912 | 58 | 1,60E-06 | K.AVGDKLPECEAVCGKPK.N |

### Example 2. Determination of the viability of embryos by measuring the human haptoglobin alpha-1 fragment

The blinded analysis that was performed after the identification of the fragment was performed on a number of 80 samples of embryos transferred on the third day. The analysis was performed on a larger number of culture medium samples, composed of samples of embryos with unknown clinical outcome (n=80) and blank control samples (n=10). Only peaks corresponding to the two adduct ions of the IS ([M+H]⁺ and [M+Na]⁺) and to the [M+8H]⁸⁺ ion of the haptoglobin alpha-1 fragment were integrated. In this phase only samples of the embryos transferred on the third day and G-1^{™}v5 controls were measured since transfer on day three is more common and the same phenomenon was observed in both sample groups (day 3 and day 5 embryos, G-1^{™}v5 and G-2^{™}v5 controls). Further it is better to assess embryo viability in the possibly earliest stage of the pre-implantation development.

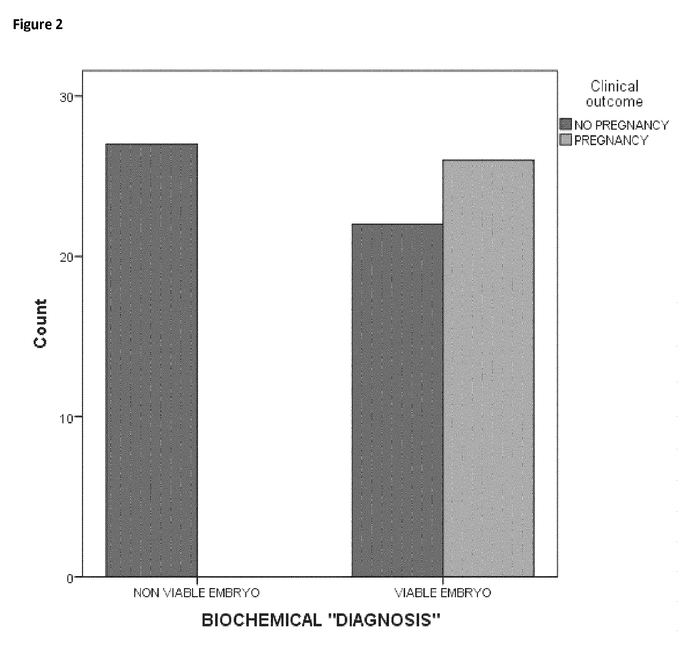

Data from this set of measurements was used parallel for two approaches, first to build a statistical database, and second to test the reliability of our method. The latter was done by the blind analysis of coded samples which was only resolved after the measurement was complete, and a biochemical "diagnosis" had been established whether the embryo possibly belonged to the viable or to the non-viable group. The values of raw peak area data of the samples were compared to the values measured in empty control medium incubated under similar circumstances as the samples containing embryos. If the peak area of the haptoglobin fragment was under the 120% value measured in the blank control, the sample was classified as viable. This threshold was established empirically. If the measured value was above the 120% of the control, the embryo was classified as non-viable. Among the 80 samples 32 were found to be non-viable by the biochemical characterization and 48 proved to be viable. From the 32 embryos classified as non-viable there was not a single case where the transfer resulted in successful delivery. In the group where the embryos were classified as viable the birth rate was 54% (Fig 2.). During our sample collection period only three pair of twins were born, in their case both transferred embryos were classified biochemically as viable.

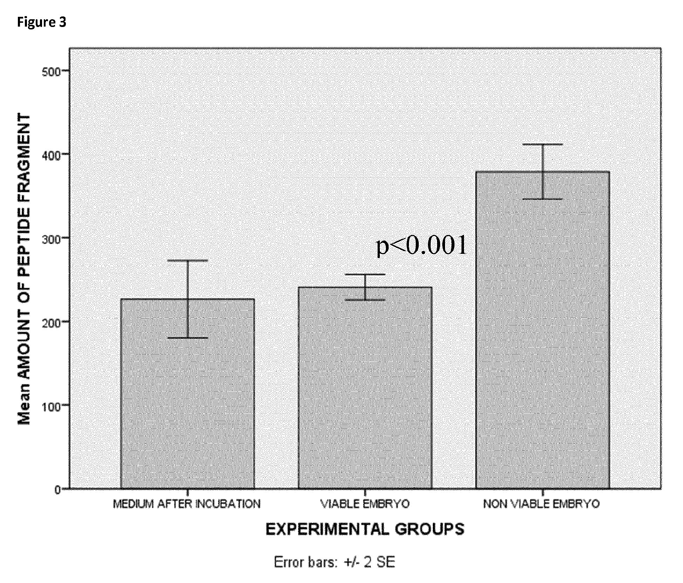

Statistical analysis of the results involved 90 embryo samples and 12 incubated blank control samples. We also investigated how different batches (n=10) of HSA affect the initial haptoglobin alpha-1 content of the culture medium. Normality of the data was tested using the one-sample Kolmogorov-Smirnov test, which revealed a normal distribution. Afterwards average values and standard deviation of the haptoglobin alpha-1 fragment amount was calculated for the controls, the different culture medium batches and all the measured embryo samples (n=90) with viable (n=55) and non-viable outcome (n=35). Correlation analysis of the amount of the peptide fragment with the diagnosis established during the blind measurements, and analysis whether significant differences exist or not between the viable embryo, non-viable embryo and blank control groups were examined using the ANOVA test. The average value of the haptoglobin fragment was 226.4 (CV 7.9%) in the control samples while 240.9 (CV 23.5%) in the samples of the viable embryos and 378.8 (CV 25.4%) in the samples of the non-viable embryos. The ANOVA test revealed a significant difference between the two embryo groups (p<0.001) and a significant correlation (p<0.001) between the amount of the peptide fragment and the pregnancy outcome. The quantified difference between these two groups was 57.2% (Fig 3.). A significant difference was also observed (p<0.001) between the incubated control medium group and the non-viable group, while there were no difference between the incubated blank control and the viable group. Blank medium samples were measured before and after incubation, but no significant difference was observed. As control however incubated blank medium samples were used.

Ten different batches of culture media were also measured after concurrent incubation under the same circumstances. These measurements were not included among the controls used in the above-described statistics. The average value of the haptoglobin alpha-1 fragment peak area in the ten media from different batches after incubation was 257.3 (CV 8.9%). The minimum peak are value was 213 while the maximum 283. (data not presented).

### Industrial applicability

The invention relates to *in vitro* methods for non-invasive embryo viability assessment during assisted reproduction. The solution is suitable to increases the pregnancies and birth rates in *in vitro* fertilization methods.

### References

1. Steptoe PC, Edwards RG. Birth after the reimplantation of a human embryo. Lancet 1978;8085:366.
2. Seli E, Sakkas D, Scott R, Kwok SC, Rosendahl SM, Burns DH. Noninvasive metabolomic profiling of embryo culture media using Raman and near-infrared spectroscopy correlates with reproductive potential of embryos in women undergoing in vitro fertilization. Fertil Steril 2007;88:1350-7.
3. de Mouzon J, Goossens V, Bhattacharya S, Castilla JA, Ferraretti AP, Korsak V et. al. European IVF-Monitoring (EIM); Consortium for the European Society on Human Reproduction and Embryology (ESHRE). Assisted reproductive technology in Europe, 2007: results generated from European registers by ESHRE. Hum Reprod 2012;27:954-66.
4. Ferraretti AP, Goossens V, de Mouzon J, Bhattacharya S, Castilla JA, Korsak V et. al. European IVF-monitoring (EIM); Consortium for European Society of Human Reproduction and Embryology (ESHRE). Assisted reproductive technology in Europe, 2008: results generated from European registers by ESHRE. Hum Reprod 2012;27:2571-84.
5. Ferraretti AP, Goossens V, Kupka M, Bhattacharya S, de Mouzon J, Castilla JA et. al. European IVF-monitoring (EIM); Consortium, for The European Society of Human Reproduction and Embryology (ESHRE). Assisted reproductive technology in Europe, 2009: results generated from European registers by ESHRE. Hum Reprod 2013;28:2318-31.
6. Corcoran D, Fair T, Lonergan P. Predicting embryo quality: mRNA expression and the preimplantation embryo. Reprod Biomed Online 2005;11:340-8.
7. Lane M, Gardner DK. Embryo culture medium: which is the best? Best Pract Res Clin Obstet Gynaecol 2007;21:83-100.
8. Jones GM, Trounson AO, Vella PJ, Thouas GA, Lolatgis N, Wood C. Glucose metabolism of human morula and blastocyst-stage embryos and its relationship to viability after transfer. Reprod Biomed Online 2001;3:124-132.
9. Hardy K, Stark J, Winston RM. Maintenance of the inner cell mass in human blastocysts from fragmented embryos. Biol Reprod 2003;68:1165-9.
10. Fancsovits P, Toth L, Takacs ZF, Murber A, Papp Z, Urbancsek J. Early pronuclear breakdown is a good indicator of embryo quality and viability. Fertil Steril 2005;84:881-7.
11. Dawson KJ, Conaghan J, Ostera GR, Winston RM, Hardy K. Delaying transfer to the third day post-insemination, to select non-arrested embryos, increases development to the fetal heart stage. Hum Reprod 1995;10(1):177-82.
12. Scott R, Seli E, Miller K, Sakkas D, Scott K, Burns DH. Noninvasive metabolomic profiling of human embryo culture media using Raman spectroscopy predicts embryonic reproductive potential: a prospective blinded pilot study. Fertil Steril 2008;90:77-83.
13. Kovalevsky G, Patrizio P. High rates of embryo wastage with use of assisted reproductive technology: a look at the trends between 1995 and 2001 in the United States. Fertil Steril 2005;84:325-30.
14. Nel-Themaat L, Nagy ZP. A review of the promises and pitfalls of oocyte and embryo metabolomics. Placenta 2011;32(Suppl 3):257S-63S.
15. Botros L, Sakkas D, Seli E. Metabolomics and its application for non-invasive embryo assessment in IVF. Mol Hum Reprod 2008;14:679-90.
16. Nagy ZP, Sakkas D, Behr B. Symposium: innovative techniques in human embryo viability assessment. Non-invasive assessment of embryo viability by metabolomic profiling of culture media ('metabolomics'). Reprod Biomed Online 2008;17:502-7.
17. Nagy ZP, Jones-Colon S, Roos P, Botros L, Greco E, Dasig J et. al. Metabolomic assessment of oocyte viability. Reprod Biomed Online 2009;18:219-25.
18. Sela R, Samuelov L, Almog B, Schwartz T, Cohen T, Amit A et. al. An embryo cleavage pattern based on the relative blastomere size as a function of cell number for predicting implantation outcome. Fertil Steril 2012;98:650-56.
19. Ng ST, Chang TH, Wu TC. Prediction of the rates of fertilization, cleavage, and pregnancy success by cumulus-coronal morphology in an in vitro fertilization program. Fertil Steril 1999;72:412-7.
20. Boiso I, Veiga A, Edwards RG. Fundamentals of human embryonic growth in vitro and the selection of high-quality embryos for transfer. Reprod Biomed Online 2002;5:328-50.
21. Magli MC, Gianaroli L, Ferraretti AP, Lappi M, Ruberti A, Farfalli V. Embryo morphology and development are dependent on the chromosomal complement. Fertil Steril 2007;87:534-41.
22. Cortezzi SS, Cabral EC, Trevisan MG, Ferreira CR, Setti AS, Braga DP et. al. Prediction of embryo implantation potential by mass spectrometry fingerprinting of the culture medium. Reproduction 2013;145:453-62.
23. Wrenzycki C, Herrmann D, Niemann H. Messenger RNA in oocytes and embryos in relation to embryo viability. Theriogenology 2007;68(Suppl 1):77S-83S.
24. Biase FH, Martelli L, Puga R, Giuliatti S, Santos-Biase WK, Fonseca Merighe GK et. al. Messenger RNA expression of Pabpnl1 and Mbd3l2 genes in oocytes and cleavage embryos. Fertil Steril 2010;93:2507-12.
25. Scott RT Jr, Ferry K, Su J, Tao X, Scott K, Treff NR. Comprehensive chromosome screening is highly predictive of the reproductive potential of human embryos: a prospective, blinded, nonselection study. Fertil Steril 2012;97:870-5.
26. Singh R, Sinclair KD. Metabolomics: approaches to assessing oocyte and embryo quality. Theriogenology 2007;68(Suppl 1):56S-62S.
27. Bromer JG, Seli E. Assessment of embryo viability in assisted reproductive technology: shortcomings of current approaches and the emerging role of metabolomics. Curr Opin Obstet Gynecol 2008;20:234-41.
28. Devreker F, Hardy K, Van den Bergh M, Winston J, Biramane J, Englert Y. Noninvasive assessment of glucose and pyruvate uptake by human embryos after intracytoplasmic sperm injection and during the formation of pronuclei. Fertil Steril 2000;73:947-54.
29. Gardner DK, Lane M, Stevens J, Schoolcraft WB. Noninvasive assessment of human embryo nutrient consumption as a measure of developmental potential. Fertil Steril 2001;76:1175-80.
30. Sturmey RG, Brison DR, Leese HJ. Symposium: innovative techniques in human embryo viability assessment. Assessing embryo viability by measurement of amino acid turnover. Reprod Biomed Online 2008;17:486-96.
31. Gada RP, Daftary GS, Walker DL, Lacey JM, Matern D, Morbeck DE. Potential of inner cell mass outgrowth and amino acid turnover as markers of quality in the in vitro fertilization laboratory. Fertil Steril 2012;98:863-9.
32. Gardner DK, Wale PL. Analysis of metabolism to select viable human embryos for transfer. Fertil Steril 2013;99:1062-72.
33. Vergouw CG, Botros LL, Judge K, Henson M, Roos P, Kostelijk EH et. al. Non-invasive viability assessment of day-4 frozen-thawed human embryos using near infrared spectroscopy. Reprod Biomed Online 2011;23:769-76.
34. Katz-Jaffe MG, Linck DW, Schoolcraft WB, Gardner DK. A proteomic analysis of mammalian preimplantation embryonic development. Reproduction 2005;130:899-905.
35. Katz-Jaffe MG, Gardner DK, Schoolcraft WB. Proteomic analysis of individual human embryos to identify novel biomarkers of development and viability. Fertil Steril 2006;85:101-7.
36. Brill A, Torchinsky A, Carp H, Toder V. The role of apoptosis in normal and abnormal embryonic development. J Assist Reprod Genet 1999;16:512-9.
37. Rosenbluth EM, Shelton DN, Wells LM, Sparks AE, Van Voorhis BJ. Human embryos secrete microRNAs into culture media-a potential biomarker for implantation. Fertil Steril 2014;101:1493-500.
38. Darcel CL, Kaldy MS. Further evidence for the heterogeneity of serum albumin. Comp Biochem Physiol B 1986;85:15-22.
39. Fasano A. Zonulin and its regulation of intestinal barrier function: the biological door to inflammation, autoimmunity, and cancer. Physiol Rev 2011;91:151-75.
40. Polticelli F, Bocedi A, Minervini G, Ascenzi P. Human haptoglobin structure and function a molecular modelling study. FEBS J 2008;275:5648-56.
41. Quinn P. Culture Media, Solutions, and Systems in Human ART Cambridge University Press, 2014.
42. Manual of Assisted Reproductive Technologies and Clinical Embryology JP Medical Ltd, 2012
43. Bungun M, Humaidan P, Bungum L. Recombinant human albumin as protein source in culture media used for IVF: a prospective randomized study Reproductive BioMedicine Online 20024 (3) 233-236
44. Wild D. The Immunoassay Handbook, Fourth Edition: Theory and applications of ligand binding, ELISA and related techniques Elsevier Science, 2013
45. Gosling JP. Immunoassays: A Practical Approach. Oxford University Press; 2000.
46. Karpova, MA, Moshkovskii, SA, Toropygin, IY and Archakov, Al, Cancer-specific MALDI-TOF profiles of blood serum and plasma: Biological meaning and perspectives. Journal of Proteomics 73 (2010) 537 - 551
47. Gebauer M, Skerra A. "Engineered protein scaffolds as next-generation antibody therapeutics". Curr Opin Chem Biol 13 (3) (June 2009) 245-255.
48. Gosling, JP Immunoassays: A Practical Approach Oxford University Press; 2000 (48, 49).
49. Ye, Bin et al., Haptoglobin- Subunit As Potential Serum Biomarker in Ovarian Cancer: Identification and Characterization Using Proteomic Profiling and Mass Spectrometry. Clinical Cancer Research, 9, (2003) 2904-2911.

## Claims

1. *In vitro* method for non-invasive embryo viability assessment comprising
a) providing a test sample of an embryo culture medium (test sample);
b) providing a sample of a control culture medium without the embryo (control sample);
both the sample and the control culture media comprising haptoglobin,
c) detecting the level of a fragment of haptoglobin in the test sample and in the control sample;
wherein a higher level of said haptoglobin fragment in the test sample compared to the control sample is indicative of an embryo in the embryo culture medium, said embryo having a limited chance for or being inappropriate for resulting in successful pregnancy.

2. The method according to claim 1, wherein the fragment of the haptoglobin is a fragment obtained by reduction of a disulphide bond between two subunits of the haptoglobin, said fragment having a sequence specific for said haptoglobin, preferably
the fragment of the haptoglobin being a haptoglobin alpha fragment, preferably a human haptoglobin alpha-1 fragment.

3. The method according to claim 1 or 2, wherein the detection is carried out by an assay method selected from the group consisting of
- mass spectrometry,
- immunoassay,
- a spectroscopic method
- a functional test,
- a sequencing method.

4. The method according to claim 3, wherein a separation step of separating said fragment from the test sample and from the control sample is carried out before the detection of the fragment.

5. The method according to any of the preceding claims, wherein
if the level of the haptoglobin fragment in the test sample is at least 120% of that of the control sample the embryo is classified non-viable, and optionally
if the level of the haptoglobin fragment in the test sample is under 120% of that of the control sample the embryo is classified viable.

6. The method according to any of the preceding claims wherein the embryo is a mammalian, preferably a human embryo and the haptoglobin is a mammalian, preferably a human haptoglobin.

7. The method according to any of the preceding claims wherein both the sample and the control culture media comprise serum albumin.

8. The method for non-invasive embryo viability assessment according to any of the preceding claims combined with the microscopic evaluation of embryo viability based on embryo morphology.

9. A method for in vitro fertilization on a patient, wherein the method comprises the embryo assessment method of any of the preceding claims, and the additional step of implanting at least one embryo into the uterus of a female patient.

10. Use of an embryo culture medium for non-invasive embryo viability assessment by measuring the level of haptoglobin fragment as defined in any of claims 1 to 8, said medium comprising haptoglobin.

11. Use of a detectable binding molecule capable of specifically binding to a fragment of a haptoglobin as defined in any of the preceding claims for non-invasive embryo viability assessment.

12. A kit for determining the level of a haptoglobin fragment as define in any of the preceding claims said kit containing an epitope-specific binding molecule for detecting the level of said fragment of haptoglobin.
